# EUROPEAN PATENT SPECIFICATION

(11) **EP 4 275 720 B1**
(45) Date of publication and mention of the grant of the patent: **24.06.2026**
(21) Application number: 22172374.5
(22) Date of filing: 09.05.2022
(51) Int. Cl.: A61M 5/31, A61M 5/32, A61M 5/34, A61M 39/10

(54) **CASTELLATED SYRINGE PRODUCT LABEL**
KRONENSPRITZENPRODUKTETIKETT
ÉTIQUETTE DE PRODUIT DE SERINGUE CRÉNELÉE

(43) Date of publication of application: 15.11.2023
(73) Proprietor: Becton, Dickinson and Company, Franklin Lakes, NJ 07417-1880 (US)
(72) Inventor: LEIBBRAND, Alfred, 38640 Claix (FR)
(74) Representative: Germain Maureau

(56) References cited:
- US-A1- 2012 029 438
- US-A1- 2012 283 645
- US-A1- 2016 143 811
- US-A1- 2021 236 736

## Description

### BACKGROUND OF THE INVENTION

### Field of the Invention

The present invention is directed to a drug delivery device assembly having an adaptor element mounted thereon and a film to engage with the adaptor to prevent its rotation relative to the rest of the drug delivery device.

### Description of Related Art

When connected to a syringe, it may be desirable to prevent a luer lock adaptor from rotating relative to the syringe. This can be accomplished by heat shrinking a label or other film to both the adaptor and they syringe, for example as set forth in U.S. Patent No. 10,661,018. However, such solutions may require heat tunnel equipment, which can increase capital costs and occupy space on a manufacturing floor. US2012283645 discloses a drug delivery device comprising a removable preventing member which prevents movement of the operating member relative to the device body. In one embodiment. US2016143811 and US2021236736 show other drug delivery devices.

Further, the application of such labels or films to a pre-filled syringe, following filling of the device, may not be desirable, as heat-sensitive compositions should not unnecessarily be exposed to heated conditions. Therefore, there exists a need in the art for a film or label to be applied to an adaptor and a syringe in order to prevent the relative rotation of the adaptor that removes or reduces the need for heat shrinking. There also exists a need to provide a more secure, anti-rotational connection between the adaptor and the syringe regardless of the application method.

### SUMMARY OF THE INVENTION

Provided herein a drug delivery device having a barrel defining an interior configured to receive a composition, the barrel having a distal tip, an adaptor including a ring extending from an inner surface and mounted onto the distal tip and having a plurality of alternating grooves and ribs extending from an outer surface, the adaptor rotatable relative to the barrel, and a film including one or more tabs extending from a surface of the film along an edge of the film, the one or more tabs corresponding to and configured to be received within one or more of the plurality of grooves, the film applied to the barrel and the adaptor such that the one or more tabs interlock with one or more of the plurality of grooves, thereby preventing relative rotation between the adaptor and the barrel.

In one configuration, the drug delivery device further includes a strip extending from the surface of the film, and wherein the one or more tabs extend from the strip. Optionally, one or more tabs are rounded. In certain configurations, the one or more tabs comprise a width and a length, and the width of the one or more tabs may correspond to a width of one or more of the plurality of grooves. Optionally, the length of the one or more tabs is at least half of a length of one or more of the plurality of grooves.

In certain configurations, the one or more tabs and the film are molded together. Optionally, the one or more tabs are molded separately from the film and affixed along an edge of the film after being molded. The one or more tabs may be co-molded with the film. In certain configurations, the syringe has a diameter of 10.85 mm.

In other configurations, the one or more tabs comprise a plurality of tabs, each of the plurality of tabs received within a groove of the plurality of grooves in the adaptor. The barrel may comprise glass, and an elastomeric stopper may be received within the interior of the barrel. The drug delivery device may further include a plug removably connected to the adaptor.

### BRIEF DESCRIPTION OF THE DRAWINGS

Figure 1 is a perspective view of a drug delivery device of an assembly together with an adaptor intended to be mounted on the distal tip of said device according to non-limiting embodiments described herein;
Figure 2 is a perspective view of a drug delivery device and adaptor according to non-limiting embodiments described herein once the adaptor is mounted on the distal tip of said device;
Figure 3 is a partial cross section view of a syringe assembly according to non-limiting embodiments described herein;
Figure 4 is a cross section view of a syringe assembly according to non-limiting embodiments described herein wherein a plug has been mounted on the adaptor;
Figure 5 is a side view of a syringe assembly according to non-limiting embodiments described herein in which the film also covers part of a plug mounted on the adaptor, said heat-shrinkable film having a dot-line as tamper evident means;
Figure 6 is a cross section view of a syringe assembly according to non-limiting embodiments described herein, onto which a connector has been partly screwed;
Figure 7 is a perspective view of a label for the assembly according to non-limiting embodiments described herein;
Figure 8 is a perspective view of the label of Figure 7, showing the label rolled up as if applied to the assembly of the present disclosure;
Figure 9 is another perspective view of a label for the assembly according to non-limiting embodiments described herein; and
Figure 10 is another perspective view of a label for the assembly according to non-limiting embodiments described herein.

### DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art. The scope of the invention is defined by the appended claims.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

It should be understood that any numerical range recited herein is intended to include all values and sub-ranges subsumed therein. For example, a range of "1 to 10" is intended to include all sub-ranges between (and including) the recited minimum value of 1 and the recited maximum value of 10, that is, having a minimum value equal to or greater than 1 and a maximum value of equal to or less than 10.

Disclosed herein are a label with one or more castellations, and a syringe assembly including such a label, as well as methods of applying such a label to a syringe assembly to prevent or limit rotation of a luer lock adaptor present on the syringe.

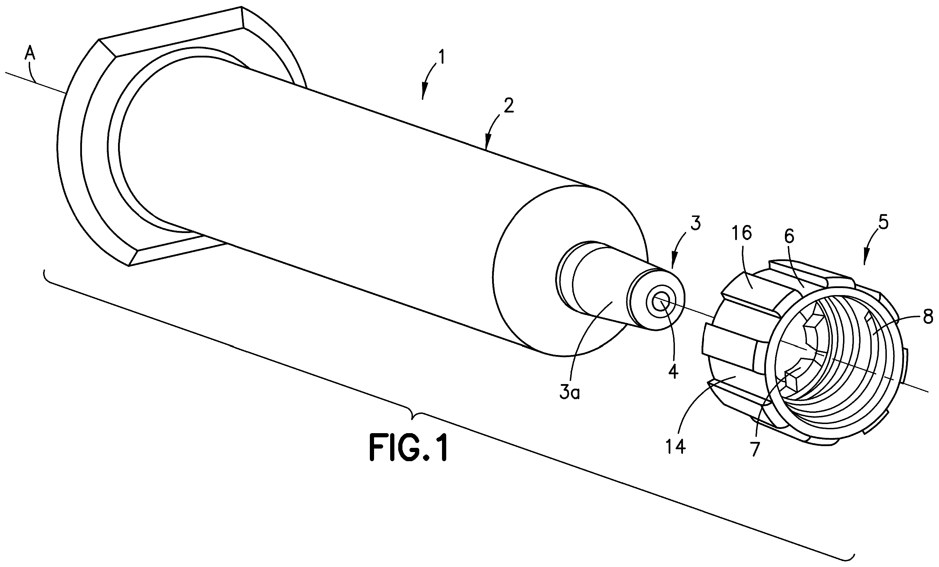

With reference to Figure 1, shown is a drug delivery device 1 of an assembly of the present disclosure. Drug delivery device 1 may include a barrel 2 defining an interior configured to hold a composition, and having a distal tip 3. Drug delivery device 1 has a longitudinal axis A. Drug delivery device 1 may be a manual syringe, optionally a pre-filled syringe. As appears on this figure, the barrel 2 and the distal tip 3 may be formed of one single element (e.g., plastic, glass, or other known materials). The barrel 2 and the distal tip 3 are preferably made of glass material. The barrel 2 may be sealed at its proximal end by a piston (not shown). The distal tip 3 encompasses a channel 4 aligned with the longitudinal axis A and providing a passageway for the transfer of the composition, either from the barrel 2 to a connector such as an IV connector or an injection needle, or from a vial to the barrel 2. On the example shown, the outer surface 3a of the distal tip 3 has a slightly tapered shape.

With continuing reference to Figure 1, also shown is an adaptor 5, said adaptor 5 including a ring 6. In the illustrated embodiment, the ring 6 is provided in its proximal region with an inner projection under the form of a discontinuous annular bulge 7 extending radially inwardly. The discontinuous annular bulge 7 may be made of a material flexible enough to allow said discontinuous annular bulge 7 to expand slightly radially in the outward direction under pressure exerted on the inner wall of said bulge 7. Alternatively, the discontinuous annular bulge may be replaced by a continuous annular bulge capable of expanding radially outwardly. The inner wall of the ring 6 is provided with an internal thread 8 distally spaced from the discontinuous annular bulge 7. The adaptor 5 is preferably made of flexible material such as plastic.

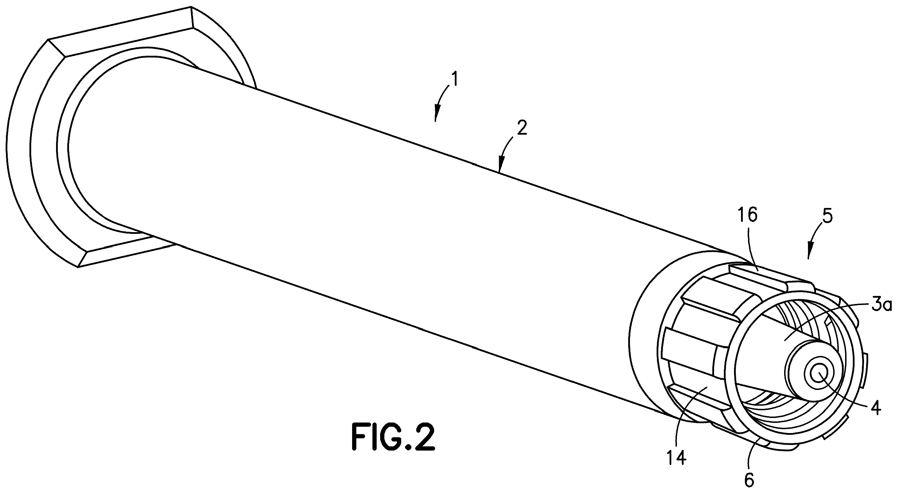

Turning to Figure 2, adaptor 5 may be mounted on the distal tip 3 by friction fit, an adhesive, a threaded connection, or other connection known to those of skill in the art.

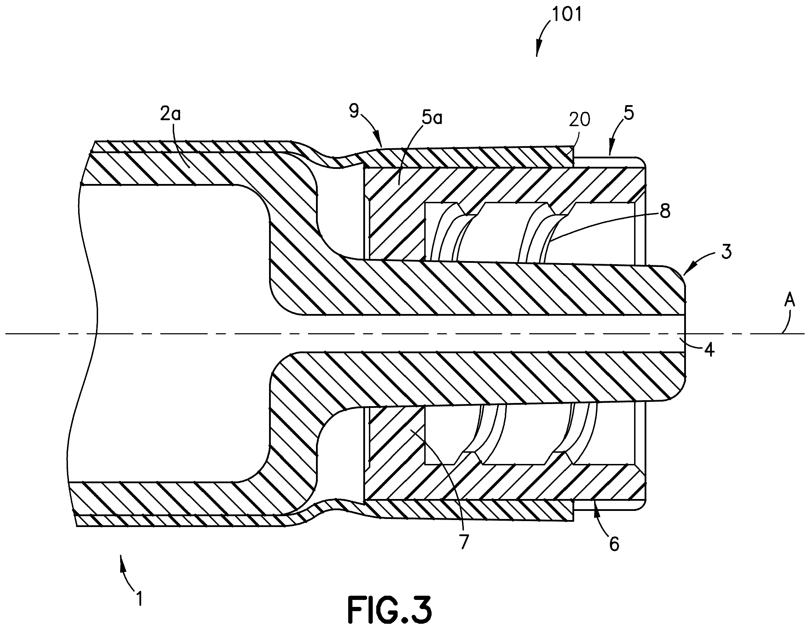

With reference to Figure 3, shown is a partial view of an assembly 101 in which the drug delivery device 1 is shown with adaptor 5 mounted on the distal tip 3. Figure 3 further shows a film 9, optionally a label including one or more indicia, that has been attached to at least a part of the barrel 2 and part of the adaptor 5. Optionally, film 9 may then be heat shrunk so as to cover and wrap tightly at least part of said barrel 2, a distal region 2a of said barrel 2 on the example shown, and at least part of said adaptor 5, a proximal region 5a of the adaptor 5 on the example shown. Film 9 may be continuous from the distal region 2a of the barrel 2 to the proximal part 5a of the adaptor 5, and may be arranged so as to cover equal amounts of barrel 2 and adaptor 5. Film 9 may be made of a thermoplastic material. In an embodiment, the film 9 is made of a polyvinyl chloride-containing material. In an embodiment, the film 9 may be transparent and/or may comprise writing such as for example graduation, brand name, lot number, expiration date, drug code, or other useful information.

As will be explained in greater detail below, as can be appreciated from Figure 3, adaptor 5 is limited and/or prevented from rotating with respect to the barrel 2 and to the distal tip 3 around the longitudinal axis A by the presence of film 9. Film 9 may also prevent the adaptor 5 from translating with respect to the barrel 2. As a result, when a user proceeds attaches drug delivery device 1 including adaptor 5 to another device or connector, the operation is facilitated and secure.

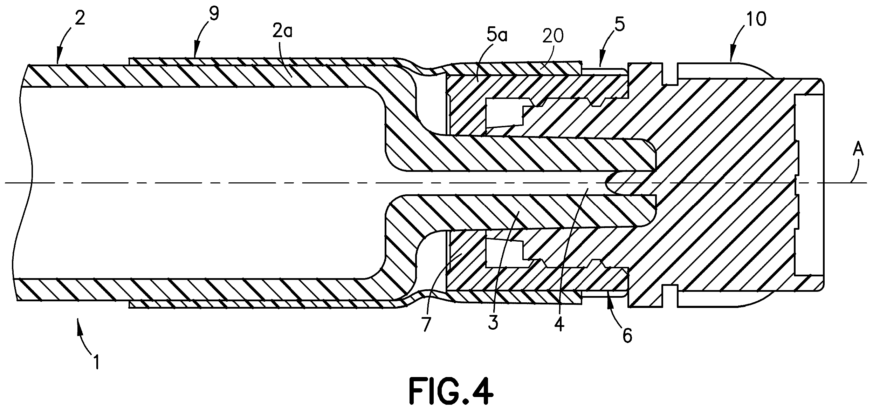

Turning to Figure 4, shown is an assembly 101 with a plug 10 mounted on the adaptor 5. This plug 10 may be removably mounted on the adaptor 5, and may limit access to the adaptor 5 before use, in order to protect said adaptor 5 and/or maintain sterility of adaptor 5.

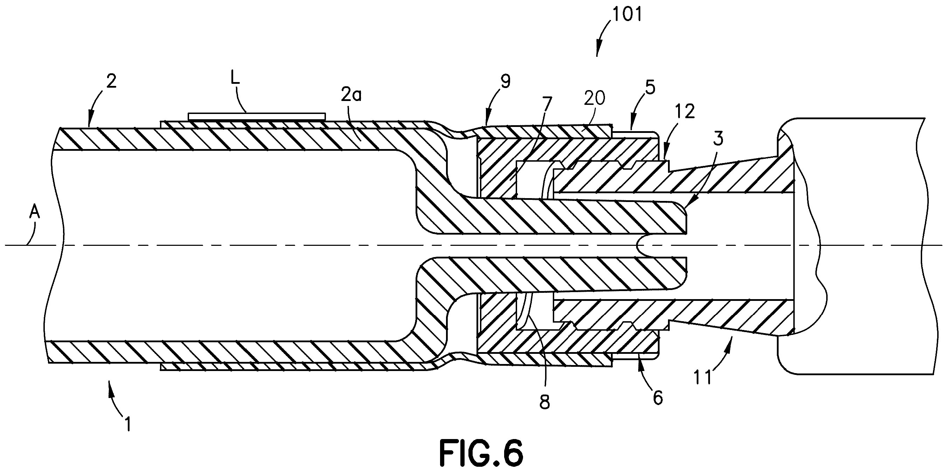

Figure 6 shows the assembly of figure 3 during the step of screwing a connector 11 on the adaptor 5. Connector 11 is provided with an outer thread 12 intended to match the inner thread 8 of the ring 6 of the adaptor 5.

Turning to Figures 4 and 6, when a user, provided with an assembly 101 with a closed adaptor 5 as shown in Figure 4, begins the process of connecting adaptor 5 to another device having a luer connection (e.g., an IV connector), such as connector 11, the user may first grasp the assembly 101 via the film 9 in one hand to remove plug 10. The user may then attach the connector 11 on to the internal thread 8 of the adaptor 5, as shown in Figure 6, without concern as to rotation of adaptor 5, or axial translation of adaptor 5, relative to barrel 2. When the external thread 12 of connector 11 is firmly received by the internal thread 8 of the adaptor 5, the user can be confident that drug delivery device 1 and the connector 11 are tightly connected without risk of leakage.

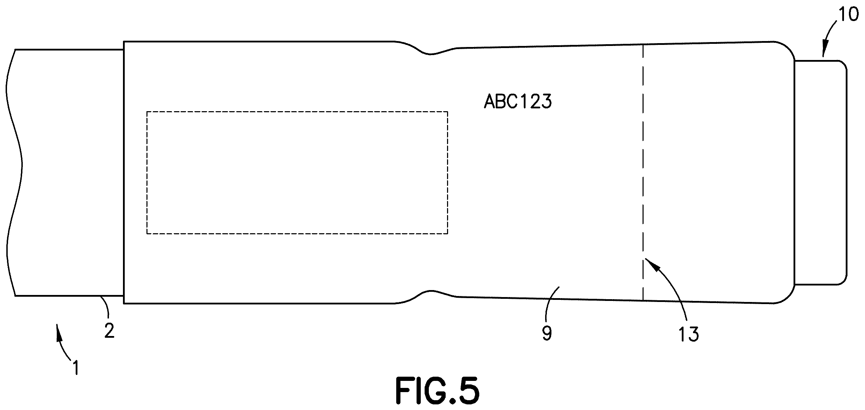

Turning to Figure 5, shown is another embodiment, in which film 9 is longer and covers at least part of plug 10. Film 9 may therefore cover part of the barrel 2, the adaptor (not shown) and part of the plug 10, optionally in a continuous manner. In such a case, film 9 may be provided with an annular breakable line 13, which must be broken in order to remove the plug 10 and open the adaptor 5. The breakable line 13 therefore may act as a tamper indicator.

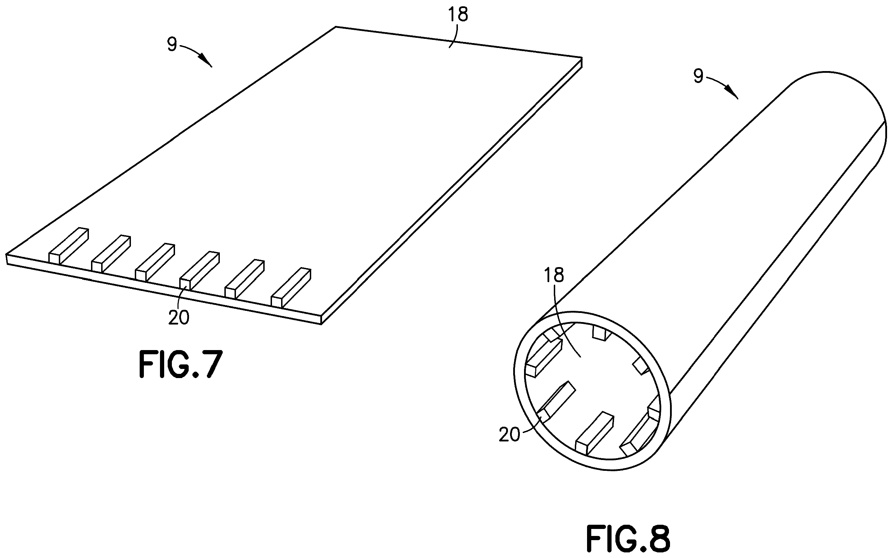

Turning to Figures 7-10, shown are an interior surface of film 9 is shown. As above, film 9 may attach to both adaptor 5 and barrel 2 in order to limit or prevent relative rotation between adaptor 5 and barrel 2. Film 9 may also include a label with indicia, as described above, providing information relevant to the contents of or use of the drug delivery device 1. As shown in Figures 1 and 2, adaptor 5 includes alternating grooves 14 and ribs 16 along its outer surface. Film 9 includes one or more cooperating castellations (e.g., tabs) 20 extending from the surface 18 that contacts the adaptor 5 and barrel 2 (e.g., inner surface of film 9). The tab(s) 20 may be placed along an edge of the film 9 and may be arranged so that they fit within one or more of the grooves 14 on the adaptor 5 when the film 9 is applied to both the barrel 2 and adaptor 5. When applied to the adaptor 5, the tab(s) 20 engage and interlock with the ribs 16, similar to locking gears on a shaft. This engagement limits and/or prevents relative rotation of the adaptor 5 given the film's connection to both the barrel 2 and the adaptor 5. The widths of the tab(s) 20 may substantially correspond to the widths of the grooves 14 to establish a strong interlocking effect. As shown, the lengths of the tab(s) 20 generally correspond to the lengths of the grooves 14, but different lengths may be used so long as the interlocking effect is maintained and relative rotation of the adaptor 5 is prevented. For example, a label 9 having a short length may be used where the label only extends partially along the adaptor 5. In this instance, the lengths of the tab(s) 20 will not necessarily correspond to the lengths of the grooves 14, and will only extend partially along the adaptor 5. Although six tabs 20 are shown in the accompanying figures, it is contemplated that any number of tabs 20 may be used to engage any number of grooves 14 depending on the design and desired strength of the interlock between the tabs 20 and adaptor 5. In some instances, only one tab 20 may be needed.

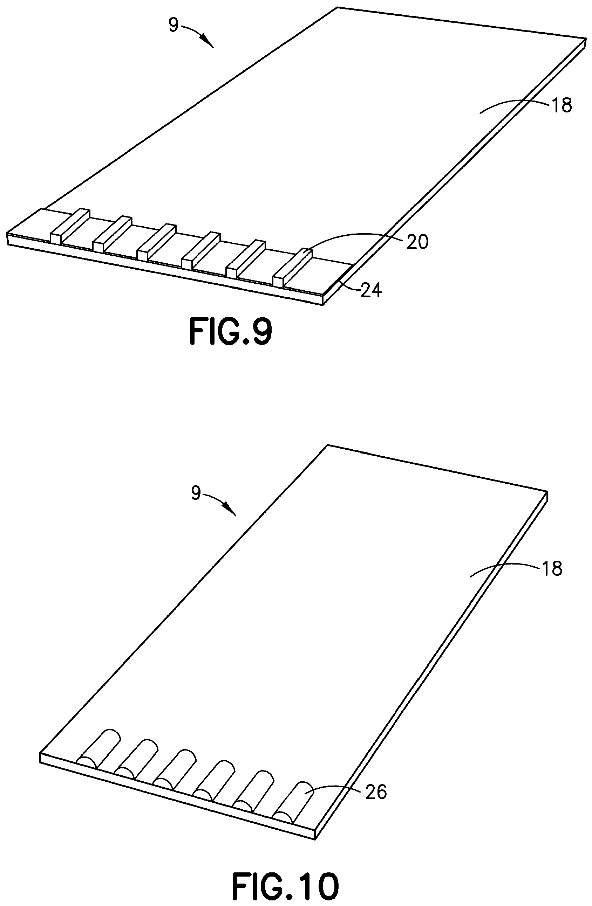

As shown in Figure 7, the tab(s) 20 are molded directly into the film 9, extending from the surface 18 and spaced apart from one another by a distance corresponding to the size of the ribs 16. Figure 8 shows the film 9 as if it were applied to a drug delivery device 1, but without the barrel 2 or adaptor 5 in place. The tabs 20 may also be co-molded along with the film 9 and later affixed thereto after the film 9 has been cut to size. Figure 9 shows another embodiment of the film 9 having a strip 24 extending from the surface 18 and along an edge of the film 9. The tabs 20 are spaced out along the strip 24 and extend therefrom in a direction away from the film 9. The strip 24 may be molded directly into the film 9, or it may be co-molded and affixed to the film 9. Figure 10 shows rounded tabs 26. The rounded tabs 26 allow for a greater clearance with the grooves 14 and permit the rounded tabs 26 to be placed slightly offset from the grooves 14 and still function as previously described. The rounded tabs 26 may also be molded directly into the film 9, or they may be co-molded and later affixed thereto. It is contemplated that the tabs 20 may take other shapes, so long as the interlocking effect between the tabs 20 and the grooves 14 is maintained.

Film 9 may be applied to the barrel 2 and adaptor 5 in the same manner as described above. Film 9 may be heat shrunk to barrel 2 and adaptor 5, or film 9 may be provided with a glue or other adhesive along the surface 18 in order to stick to the barrel 2 and to lock adaptor 5. Film 9 may also be used in connection with a plug 10 in a manner described above. Given the diameter of the adaptor 5, the film may be used on syringes with a diameter of approximately 10.85 millimeters.

The assembly may be manufactured according to the following: a drug delivery device 1 including a barrel 2 for a composition, such as a pharmaceutical composition, may be provided. Barrel 2 may include a distal tip 3 encompassing a channel providing a passageway for the transfer of the composition, and a suitable adaptor 5. In a second step, the adaptor 5 is connected to distal tip 3 of barrel 2, for example by friction fit. Barrel 2 may optionally be filled with a composition before this second step, or after this second step. In a further step, film 9 is attached to at least a portion of barrel 2 and in order to lock adaptor 5 relative to barrel 2, with alignment between one or more tab(s) 20 and one or more groove(s) 14 in adaptor 5, for example by heat shrinking, an adhesive, or other like method.

In the non-limiting embodiment in which film 9 also covers part of a plug 10 mounted on adaptor 5, plug 10 may be mounted on the adaptor 5 before bonding the film 9, and the length of the shrinkable film is chosen so as to cover the adaptor 5 and at least part of plug 10, optionally in a continuous way as described above (with or without optional breakable lines as described above). In such embodiments, one or more tab(s) 20 are arranged longitudinally on label 9 so as to still align, and be received by, one or more groove(s) 14 in adaptor 5.

Although the present disclosure has been described in detail for the purpose of illustration based on what is currently considered to be the most practical and preferred embodiments, it is to be understood that such detail is solely for that purpose and that the present disclosure is not limited to the disclosed embodiments, but, on the contrary, is intended to cover modifications and equivalent arrangements as long as they are within the scope of the appended claims. For example, it is to be understood that the present disclosure contemplates that, to the extent possible, one or more features of any embodiment can be combined with one or more features of any other embodiment.

## Claims

1. A drug delivery device (1) comprising:
a barrel (2) defining an interior configured to receive a composition, the barrel (2) comprising a distal tip (3) defining a channel (4) to provide a passageway for transfer of the composition;
an adaptor (5) comprising a ring (6) extending from an inner surface and mounted onto the distal tip (3) and comprising a plurality of alternating grooves (14) and ribs (16) extending from an outer surface, the adaptor rotatable relative to the barrel (2); and
a film (9) configured to be applied to the barrel (2) and the adaptor (5) for preventing relative rotation between the adaptor (5) and the barrel (2),
**characterized in that** the film (9) comprises one or more tabs (20) extending from a surface (18) of the film (9) along an edge of the film (9), the one or more tabs (20) corresponding to and configured to be received within one or more of the plurality of grooves (14),
wherein the film (9) is configured to be applied to the barrel (2) and the adaptor (5) such that the one or more tabs (20) interlock with one or more of the plurality of grooves (14), thereby preventing relative rotation between the adaptor (5) and the barrel (2).

2. The drug delivery device (1) of claim 1, further comprising a strip (24) extending from the surface (18) of the film, and wherein the one or more tabs (20) extend from the strip (24).

3. The drug delivery device (1) of claim 1 or claim 2, wherein the one or more tabs (20) are rounded.

4. The drug delivery device (1) of any of claims 1-3, wherein the one or more tabs (20) comprise a width and a length.

5. The drug delivery device (1) of claim 4, wherein the width of the one or more tabs (20) corresponds to a width of one or more of the plurality of grooves (14).

6. The drug delivery device (1) of claim 4, wherein the length of the one or more tabs (20) is at least half of a length of one or more of the plurality of grooves (14).

7. The drug delivery device (1) of any of claims 1-6, wherein the one or more tabs (20) and the film (9) are molded together.

8. The drug delivery (1) device of any of claims 1-7, wherein the one or more tabs (20) are molded separately from the film (9) and affixed along an edge of the film (9) after being molded.

9. The drug delivery device (1) of any of claims 1-7, wherein the one or more tabs (20) are co-molded with the film (9).

10. The drug delivery device (1) of any of claims 1-9 wherein the syringe has a diameter of 10.85 mm.

11. The drug delivery device (1) of any of claims 1-10, wherein the one or more tabs (20) comprise a plurality of tabs (20), each of the plurality of tabs (20) received within a groove (14) of the plurality of grooves (14) in the adaptor (5).

12. The drug delivery device (1) of any of claims 1-11, wherein the barrel (2) comprises glass.

13. The drug delivery device (1) of any of claims 1-12, further comprising an elastomeric stopper received within the interior of the barrel (2).

14. The drug delivery device (1) of any of claims 1-13, further comprising a plug (10) removably connected to the adaptor (5).

## Patentansprüche

1. Arzneimittelabgabevorrichtung (1) umfassend:
einen Zylinder (2), der einen Innenraum definiert, der konfiguriert ist, um eine Zusammensetzung aufzunehmen, wobei der Zylinder (2) eine distale Spitze (3) umfasst, die einen Kanal (4) definiert, um einen Durchgang für den Transfer der Zusammensetzung bereitzustellen;
einen Adapter (5), der einen Ring (6) umfasst, der sich von einer Innenoberfläche erstreckt und an der distalen Spitze (3) montiert ist und eine Vielzahl von abwechselnden Nuten (14) und Rippen (16) umfasst, die sich von einer Außenoberfläche erstrecken, wobei der Adapter in Bezug zum Zylinder (2) drehbar ist; und
eine Folie (9), die konfiguriert ist, um auf den Zylinder (2) und den Adapter (5) aufgebracht zu werden, um eine relative Drehung zwischen dem Adapter (5) und dem Zylinder (2) zu verhindern,
**dadurch gekennzeichnet, dass** die Folie (9) eine oder mehrere Laschen (20) umfasst, die sich von einer Oberfläche (18) der Folie (9) entlang eines Rands der Folie (9) erstrecken, wobei die eine oder mehrere Laschen (20) einer oder mehreren der Vielzahl von Nuten (14) entsprechen und konfiguriert sind, um darin aufgenommen zu werden,
wobei die Folie (9) konfiguriert ist, um auf den Zylinder (2) und den Adapter (5) aufgebracht zu werden, sodass die eine oder mehrere Laschen (20) mit einer oder mehreren der Vielzahl von Nuten (14) ineinandergreifen, wodurch eine relative Drehung zwischen dem Adapter (5) und dem Zylinder (2) verhindert wird.

2. Arzneimittelabgabevorrichtung (1) nach Anspruch 1, weiter umfassend einen Streifen (24), der sich von der Oberfläche (18) der Folie erstreckt, und wobei sich die eine oder mehrere Laschen (20) von dem Streifen (24) erstrecken.

3. Arzneimittelabgabevorrichtung (1) nach Anspruch 1 oder 2, wobei die eine oder mehrere Laschen (20) abgerundet sind.

4. Arzneimittelabgabevorrichtung (1) nach einem der Ansprüche 1-3, wobei die eine oder mehrere Laschen (20) eine Breite und eine Länge umfassen.

5. Arzneimittelabgabevorrichtung (1) nach Anspruch **4,** wobei die Breite der einen oder mehrerer Laschen (20) einer Breite von einer oder mehreren der Vielzahl von Nuten (14) entspricht.

6. Arzneimittelabgabevorrichtung (1) nach Anspruch **4,** wobei die Länge der einen oder mehreren Laschen (20) mindestens der Hälfte einer Länge einer oder mehrerer der Vielzahl von Nuten (14) entspricht.

7. Arzneimittelabgabevorrichtung (1) nach einem der Ansprüche 1-6, wobei die eine oder mehrere Laschen (20) und die Folie (9) zusammengeformt sind.

8. Arzneimittelabgabevorrichtung (1) nach einem der Ansprüche 1-7, wobei die eine oder mehrere Laschen (20) getrennt von der Folie (9) geformt, und nach dem Formen entlang eines Rands der Folie (9) fixiert sind.

9. Arzneimittelabgabevorrichtung (1) nach einem der Ansprüche 1-7, wobei die eine oder mehrere Laschen (20) mit der Folie (9) zusammengeformt sind.

10. Arzneimittelabgabevorrichtung (1) nach einem der Ansprüche 1-9, wobei die Spritze einen Durchmesser von 10,85 mm aufweist.

11. Arzneimittelabgabevorrichtung (1) nach einem der Ansprüche 1-10, wobei die eine oder mehrere Laschen (20) eine Vielzahl von Laschen (20) umfassen, wobei jede der Vielzahl von Laschen (20) innerhalb einer Nut (14) der Vielzahl von Nuten (14) im Adapter (5) aufgenommen ist.

12. Arzneimittelabgabevorrichtung (1) nach einem der Ansprüche 1-11, wobei der Zylinder (2) Glas umfasst.

13. Arzneimittelabgabevorrichtung (1) nach einem der Ansprüche 1-12, weiter umfassend einen Elastomerstopfen, der im Inneren des Zylinders (2) aufgenommen ist.

14. Arzneimittelabgabevorrichtung (1) nach einem der Ansprüche 1-13, weiter umfassend einen mit dem Adapter (5) abnehmbar verbundenen Stopfen (10).

## Revendications

1. Dispositif d'administration de médicament (1) comprenant :
un cylindre (2) définissant une partie intérieure configurée pour recevoir une composition, le cylindre (2) comprenant une pointe distale (3) définissant un canal (4) afin de fournir un passage pour le transfert de la composition ;
un adaptateur (5) comprenant une bague (6) s'étendant depuis une surface interne et montée sur la pointe distale (3), et comprenant une pluralité de rainures (14) et de nervures (16) alternées s'étendant depuis une surface externe, l'adaptateur pouvant tourner par rapport au cylindre (2) ; et
un film (9) configuré pour être appliqué sur le cylindre (2) et l'adaptateur (5) afin d'empêcher une rotation relative entre l'adaptateur (5) et le cylindre (2),
**caractérisé en ce que** le film (9) comprend une ou plusieurs languettes (20) s'étendant depuis une surface (18) du film (9) le long d'un bord du film (9), la ou les plusieurs languettes (20) correspondant à une ou plusieurs rainures de la pluralité de rainures (14) et étant configurées pour être reçues dans celles-ci,
dans lequel le film (9) est configuré pour être appliqué sur le cylindre (2) et l'adaptateur (5) de sorte que la ou les plusieurs languettes (20) s'emboîtent avec une ou plusieurs rainures de la pluralité de rainures (14), empêchant ainsi toute rotation relative entre l'adaptateur (5) et le cylindre (2).

2. Dispositif d'administration de médicament (1) selon la revendication 1, comprenant en outre une bande (24) s'étendant depuis la surface (18) du film, et dans lequel la ou les plusieurs languettes (20) s'étendent depuis la bande (24).

3. Dispositif d'administration de médicament (1) selon la revendication 1 ou la revendication 2, dans lequel la ou les plusieurs languettes (20) sont arrondies.

4. Dispositif d'administration de médicament (1) selon l'une des revendications 1 à 3, dans lequel la ou les plusieurs languettes (20) présentent une largeur et une longueur.

5. Dispositif d'administration de médicament (1) selon la revendication 4, dans lequel la largeur de la ou des plusieurs languettes (20) correspond à la largeur d'une ou de plusieurs rainures de la pluralité de rainures (14).

6. Dispositif d'administration de médicament (1) selon la revendication 4, dans lequel la longueur de la ou des plusieurs languettes (20) est au moins égale à la moitié de la longueur d'une ou de plusieurs rainures de la pluralité de rainures (14).

7. Dispositif d'administration de médicament (1) selon l'une des revendications 1 à 6, dans lequel la ou les plusieurs languettes (20) et le film (9) sont moulés ensemble.

8. Dispositif d'administration de médicament (1) selon l'une des revendications 1 à 7, dans lequel la ou les plusieurs languettes (20) sont moulées séparément du film (9) et fixées le long d'un bord du film (9) après avoir été moulées.

9. Dispositif d'administration de médicament (1) selon l'une des revendications 1 à 7, dans lequel la ou les plusieurs languettes (20) sont co-moulées avec le film (9).

10. Dispositif d'administration de médicament (1) selon l'une des revendications 1 à 9, dans lequel la seringue a un diamètre de 10,85 mm.

11. Dispositif d'administration de médicament (1) selon l'une des revendications 1 à 10, dans lequel la ou les plusieurs languettes (20) comprennent une pluralité de languettes (20), chacune de la pluralité de languettes (20) étant reçue dans une rainure (14) de la pluralité de rainures (14) dans l'adaptateur (5).

12. Dispositif d'administration de médicament (1) selon l'une des revendications 1 à 11, dans lequel le cylindre (2) comprend du verre.

13. Dispositif d'administration de médicament (1) selon l'une des revendications 1 à 12, comprenant en outre un obturateur en élastomère reçu dans la partie intérieure du cylindre (2).

14. Dispositif d'administration de médicament (1) selon l'une des revendications 1 à 13, comprenant en outre un bouchon (10) relié de manière amovible à l'adaptateur (5).
